# EUROPEAN PATENT APPLICATION

(11) **EP 4 169 435 A1**
(43) Date of publication of application: **26.04.2023**
(21) Application number: 22202412.7
(22) Date of filing: 19.10.2022
(51) Int. Cl.: A61B 1/00, A61M 25/00, G02B 23/24

(54) **METHODS OF PRODUCING A FLEXIBLE SPIRAL MEDICAL TUBE**

(30) Priority: 20.10.2021 PL 43926621
(71) Applicant: Extrudan Sp. z o.o., 62-070 Dabrowka (PL)
(72) Inventor: Pedersen, John-Eric, 61-854 Poznan (PL)
(74) Representative: Kondrat, Mariusz

(57) **Abstract**

Invention relates to a method of producing a flexible spiral medical tube consisting of a tubular portion and a braid, characterised in that it comprises the following steps: a) filling a first extruder with a first material and a second extruder with a second material having a hardness greater than the first material; b) mixing the materials in one mixing head (8) connected to the first and the second extruder; c) forming a first material tape (5) and a second material roll (6) in the mixing head (8) of the extruders; d) obtaining a tape with an embedded roller (4) by extruding both materials through a shaped mouthpiece (9); e) winding the tape with the embedded roller (4) obtained in step d) on a rod (7) fixed in the gear (10), which is a spiral grooved rod, in recesses of which steel flexible rollers (12) are guided, or on a circular cross-section securing rod containing substantially cruciform guide sleeves (13) equipped with recesses for guiding flexible steel rollers (12), wherein the flexible rollers (12), transmitting the torsional moment from a motor of a gear (10) simultaneously push the wound tape with the embedded roller (4) in the axial direction, creating a spiral, wherein the edges of the tape with the embedded roller (4) are overlapped and fused together, and while the tape with the embedded roller (4) is wound on the rod (7), a curing of the produced tube (1) is carried out, which then slid off the rod (7). In another aspect, the invention relates to a method of producing a flexible spiral medical tube consisting of a tubular portion and a braid, characterised in that the materials are mixed in two mixing heads and extruded through two mouthpieces (9a, 9b), wherein the tape (5) of the first material is extruded through first mouthpiece (9a) with a shape of the tape (5) cross-section, and the roller (6) of the second material is extruded through a shaped mouthpiece of a second mixing head (9b) with a shape of the roller cross-section; the tape (5) connects to the roller (6) by fusing the roller into the tape (5) while being wound onto the rod (7). In another aspect, the invention relates to a flexible spiral medical tube consisting of a tubular portion and a braid, characterised in that its internal diameter < 10 mm; the braid (3) is fused into the tubular portion (2) over its entire contact surface with the tubular portion (2); the tubular portion (2) is made of the first material and the braid (3) is made of the second material; wherein the hardness of the second material is higher than that of the first material.

## Description

The subject of the invention is a flexible spiral medical tube consisting of a tubular part and a braid, and the methods of its production.

In the single-use medical products sector, the most important features are lack of chemical and biological reactivity, sterility and reliability. Then, the functionality of the product in a given application should be considered. In this context, spiral medical tubes and guides are an alternative to traditional tubes.

Document EP1576980 B1 discloses a medical guide wire in which a flexible elongated core member has a reduced diameter head end portion and a hand access portion larger than the diameter of said reduced diameter head end portion, and a spiral spring is attached at both ends to said reduced diameter head end portion. Wherein said reduced diameter head end portion has a progressively decreasing diameter towards the head end of said reduced diameter head end portion or is tapered towards said head end of said reduced diameter head end portion. The disclosed medical guide wire is characterised in that the inner circumference of said spiral spring and the outer circumference of said core member are secured by a plurality of attachment points aligned in a longitudinal direction with predetermined spans, and said spiral spring and said core member are spaced apart to form a non-integral zone, wherein they are not attached to one another, the zone extending at least 20 mm and extending to said head end of said reduced diameter head end portion from the closest attachment point.

Application US4495134 A discloses a method for manufacturing a flexible tube for use in an endoscope, which comprises a basic tubular core structure including at least an inner metallic tubular spiral having an outer side and an outer meshwork tube covering the outer side of the metallic tubular spiral. The disclosed method comprising the following steps: (a) locating a preformed homogeneous thermoplastic synthetic resin tube over the outer surface of the meshwork tube with a contact pressure therebetween to form an intermediate product by the steps of temporarily closing the hollow interior of the basic tubular core structure; positioning a forcibly enlarged open end of the homogeneous thermoplastic synthetic resin tube over one end of the basic tubular core structure; inflating the thermoplastic synthetic resin tube by supplying compressed gas into the interior thereof until the inner diameter of the inflated thermoplastic synthetic resin tube corresponds to the outer diameter of the basic tubular core structure; feeding the thermoplastic synthetic resin tube axially over the basic tubular core structure; reducing the internal overpressure in the interior of the thermoplastic synthetic resin tube after a desired length of said thermoplastic synthetic resin tube has been fed over the basic tubular core structure whereupon a contact pressure exists between the thermoplastic synthetic resin tube and the tubular core structure resulting from the inherent contractive forces tending to restore the synthetic resin tube to its unstressed condition; and (b) treating the intermediate product so that the thermoplastic synthetic resin of the thermoplastic synthetic resin tube partially flows into the mesh of the meshwork tube by the step of heating the thermoplastic synthetic resin tube to a temperature higher than a softening point of the thermoplastic synthetic resin forming the same, whereupon the thermoplastic synthetic resin protrudes into the mesh of the meshwork tube and so that upon plasticising the resin tube forms a coating layer bonded integrally to the basic tubular core structure.

Document US6206824 B1 discloses a flexible tube for an endoscope comprising a spiral coil defining gaps between adjacent turns of said spiral coil; and a jacket formed by a jacket material and covering at least an outer surface of said spiral coil; wherein said jacket material penetrates through said gaps to cover at least edges of said spiral coil and wherein said spiral coil includes one or more superposed layers which are covered with one or more layers of a reticulate tube, wherein said reticulate tube is embedded in said jacket material. Further, the document also discloses a method of producing said flexible tube for an endoscope comprising in sequence the steps of: setting a spiral coil as an innermost layer on a mandrel with a clearance between an outer surface of said mandrel and an inner surface of said spiral coil; applying a melt of a jacket material onto said spiral coil to penetrate through gaps between adjacent turns of said spiral coil into said clearance; curing said jacket material; and removing said mandrel. In a preferred embodiment, the bond enhancing treatment comprises one of the following: coating, plating, primer coating and adhesive application. Moreover, in a preferred embodiment, the jacket material is a thermoplastic synthetic resin and is applied with an extrusion moulding machine.

The aim of the invention is to develop a modified single-use spiral medical tube increasing the comfort of the patient and medical staff and reducing the amount of generated waste.

One aspect of the invention is a method of producing a flexible spiral medical tube consisting of a tubular portion and a braid, characterised in that it comprises the following steps:
a) filling a first extruder with a first material and a second extruder with a second material having a hardness greater than the first material;
b) mixing the materials in one mixing head connected to the first and the second extruder;
c) forming a first material tape and a second material roll in the mixing head of the extruders;
d) obtaining a tape with an embedded roller by extruding both materials through a shaped mouthpiece;
e) winding the tape with the embedded roller obtained in step d) on a rod fixed in a gear, which is a spirally grooved rod, in recesses of which steel flexible rollers are guided, or on a circular cross-section securing rod containing substantially cruciform guide sleeves equipped with recesses for guiding flexible steel rollers, wherein the flexible rollers, transmitting the torsional moment from a motor of the gear and simultaneously push the wound tape with the embedded roller in axial direction, creating a spiral, wherein the edges of the tape are overlapped and fused together, and while the tape with an embedded roller is wound on the rod, a curing of the produced tube is carried out, which then slid off the rod.

Preferably in step e) a spirally grooved rod with an almost cruciform cross-section is used.

Preferably, in step e), during the winding of the tape with an embedded roller on a rod, the distances between the turns of the braid and the place of winding the tape with an embedded roller relative to the mouthpiece of the extruder head is controlled by a profiled wheel applied to the braid.

Another aspect of the invention is a method of producing a flexible spiral medical tube consisting of a tubular portion and a braid, characterised in that it comprises the following steps:
a) filling a first extruder with a first material and a second extruder with a second material having a hardness greater than the first material;
b) extruding a tape of the first material through a shaped mouthpiece of a first mixing head with a shape of the tape cross-section and extruding a roller of the second material by extruding through a shaped mouthpiece of a second mixing head with a shape of the roller cross-section;
c) winding the tape and the roller obtained in step d) on a rod fixed in a gear, which is a spirally grooved rod, in the recesses of which steel flexible rollers are guided, or on a circular cross-section securing rod containing substantially cruciform guide sleeves equipped with recesses for guiding flexible steel rollers, while when being wound on the rod, the tape connects to the roller by fusing the roller into the tape, while flexible steel rollers transmit the torsional moment from a motor of the gear and simultaneously push the wound tape with the embedded roller in the axial direction, creating a spiral, the edges of the tape are overlapped and fused together, and while the tape is wound on the rod with an embedded roller, a curing of the produced tube is carried out, which then slid off the rod.

Preferably, the first mixing head and the second mixing head share a common housing.

Preferably in step c) a spirally grooved rod with an almost cruciform cross-section is used.

Preferably, in step c), during the winding of the tape and the roller on a rod, with the simultaneous fusion of the roller into the tape, the distances between the turns of the braid and the place of winding the tape with an embedded roller relative to the mouthpiece is controlled by a profiled wheel applied to the braid.

Another aspect of the invention is a flexible spiral medical tube consisting of a tubular portion and a braid, characterised in that its internal diameter < 10 mm; the braid is fused into the tubular portion over its entire contact surface with the tubular portion; the tubular portion is made of a first material and the braid is made of a second material; wherein the hardness of the second material is higher than that of the first material.

Preferably, the inner diameter of the tube according to the invention is 6 mm.

Preferably, the first material is selected from the group consisting of polyvinyl chloride (PVC), thermoplastic elastomers (TPE), polyethylene (PE).

Preferably, the first material is selected from the group consisting of polyvinyl chloride (PVC), thermoplastic elastomers (TPE), polyethylene (PE).

Preferably, the braid pitch is constant.

The invention provides the following advantages:
- provides a thin-walled spiral medical tube with parameters previously unavailable on the market, i.e. with a reduced weight and an internal diameter of <10 mm;
- provides increased comfort for the patient and medical staff. Patients often "connected" with heavy tubes to life-saving medical equipment experience great discomfort, and similarly, medical personnel who find it difficult to manipulate these tubes;
- the tube according to the invention may find wide application in medicine, e.g. as a line for the administration of fluids or gases during surgical procedures or as part of equipment used in the care of patients in hospitals and nursing homes;
- smaller volume and weight parameters of the tube according to the invention ensure a significant reduction in the amount of generated waste (which is particularly significant in the case of disposable medical products made of plastics, which are toxic waste intended for incineration);
- smaller volume and weight parameters of the tube according to the invention provide a significant reduction in energy expenditure for transport - lower cost of transporting packages with lower weight.

The invention is illustrated in embodiments and in the drawing in which, Fig. 1 illustrates the medical spiral tube of the invention in a pictorial view, in cross section and in section A-A; Fig. 2 illustrates a tape with an embedded roller constituting an intermediate product in the method of the invention, wherein the tape is shown in a pictorial view and in a cross-section; Fig. 3 illustrates a spirally grooved rod; Fig. 4 illustrates the winding of the tape with the embedded roller on a spirally grooved rod in a method step according to the invention; Fig. 5 illustrates a profiled wheel; Fig. 6 illustrates an embodiment of a grooved rod with steel flexible rollers in cross section; Fig. 7 illustrates a side view of an embodiment of a grooved rod with steel flexible rollers; Fig. 8 illustrates a guide sleeve; Fig. 9 shows the rod in a variant of a typical circular rod on which a plurality of guide sleeves is applied to guide steel flexible rollers; Fig. 10 shows the separate winding of the tape and the roller onto the rod.

### Example 1.

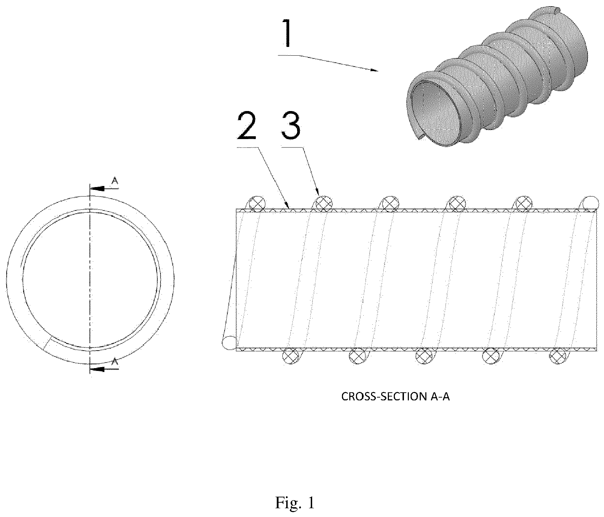

In this embodiment, for the production of a flexible spiral medical tube **1** according to the invention illustrated in Fig. 1, two extruders (constituting devices known from the state of the art) were used, loaded with two different materials, including the first material which was intended to produce the tubular portion **2** of the tube **1,** and the second material which was intended to produce the spiral braid **3.** Wherein, in accordance with the invention, the second used material has a higher hardness than the first material.

In this embodiment, said extruders are two "Golden Conch/Zhoushan" extruders characterised in that:
- these are single-screw extruders with a monolithic screw and a straight cylinder
- the feed hopper is adapted to work with granulate, regrind or powder
- they are adapted to the processing of a wide range of plastics (including polyvinyl chloride (PVC), polyethylene (PE), thermoplastic elastomers (TPE))
- drives are based on Alternating Current motors regulated with power inverters
- have helical gears with a ratio of 1:55
- they have the system for cylinder plasticisation into 3 zones with independently regulated temperature (each portion has a separate temperature controller and a cooling fan), which ensures that the processed material obtains the correct temperature and density, and thus its effective plasticisation.

Detailed parameters of the extruders are presented in Table 1:

**Table 1: Parameters of the extruder for the tape and for the roller from which the braid is formed**

| Feature | Extruder for the tape | Extruder for the roller |
|---|---|---|
| screw diameter [mm] | 45 | 38 |
| L/D | 28 | 28 |
| Motor power [kW] | 11 | 7.5 |
| Efficiency [kg/h] | 2.5 | 2 |

In this embodiment, the first material for the production of the tubular portion **2** was polyvinylchloride (PVC) "738", while other materials, e.g. thermoplastic elastomers (TPE) or polyethylene (PE), can be used to produce the tubular part. In this embodiment, the second material for the production of the spiral braid **3** was polyvinylchloride (PVC) "918", while other materials, e.g. thermoplastic elastomers (TPE) or polyethylene (PE), can be used to produce the tubular part.

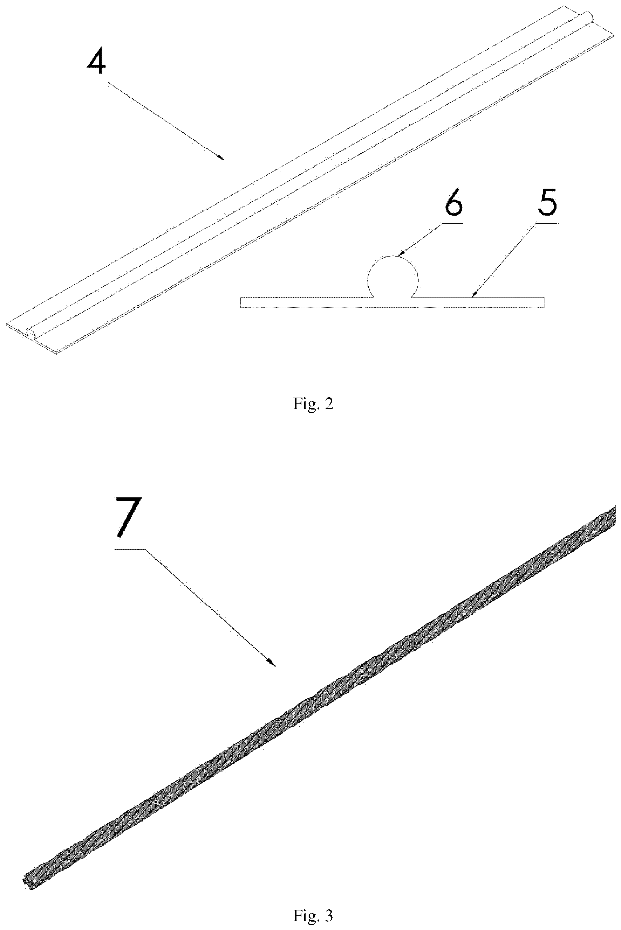

Both materials were then mixed in the mixing head **8** of a special construction of the extruders. Moreover, in this embodiment, the mixing head **8** is connected to two extruders, each of which being loaded with the same type of material with different hardness. In the mixing head, a flat and thin tape **5** of the first material (lower hardness) and the roller **6** of the second material (higher hardness) are formed. Both of these parts (i.e. the tape **5** and the roller **6)** are connected during the extrusion by the shaped mouthpiece **9** with a shape of tape cross-section with the embedded roller **4.** As a result of the extrusion, a flat and thin tape **5** of the first material with the embedded roller **6** of the second material, from which the braid **3** will be formed, are produced. The appearance of the tape with the embedded roller **4** consisting of the flat and thin tape **5** and the roller **6** is illustrated in Fig. 2.

Then, the tape obtained in this way with the embedded roller **4** is wound onto a spirally grooved rod **7** with a cross-section similar to the shape of a cross, which is shown in Fig. 3. Wherein, steel flexible rollers **12** are guided in the spiral grooves (recesses) of the spirally grooved rod **7,** and, thanks to their structure, transfer the torsional moment from the motor of the gear **10.** In said gear **10** said spirally grooved rod **7** and steel flexible rollers **12** are fastened.

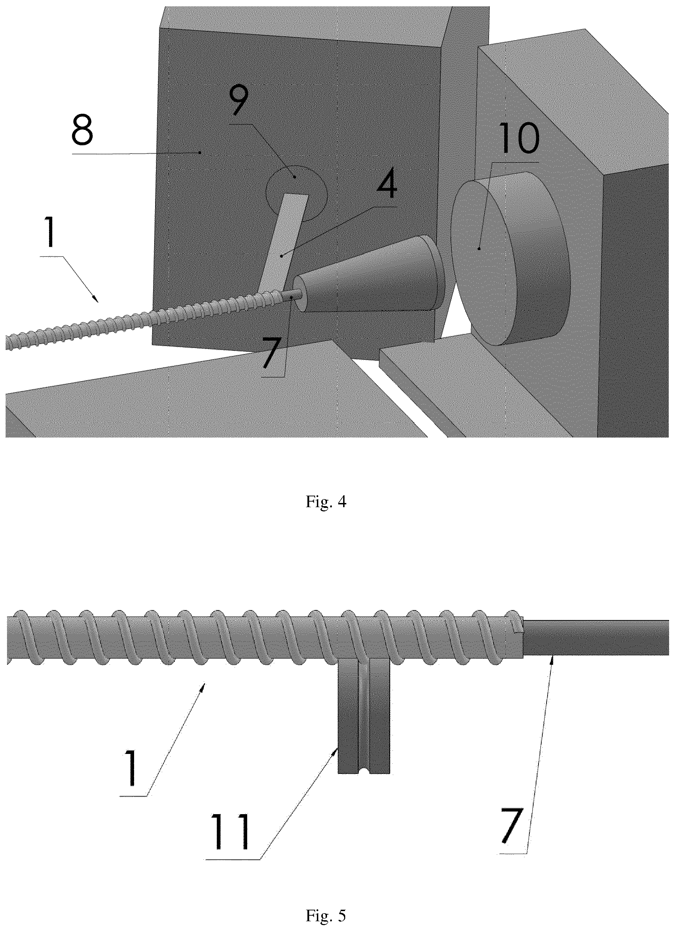

The rotation of these steel flexible rollers **12** causes the tape with embedded roller **4** from the mouthpiece **9** of the extruder head **8** of the extruders to be simultaneously wound on the rod **7** and pushed in the axial direction, which creates a spiral. One edge of the tape with the embedded roller **4** is wound "overlapping" on the other edge and they are fused together. At this stage, the materials forming the tube **1** are hot and plastic. The step of winding the tape with the embedded roller **4** on a spirally grooved rod **7** is presented in Fig. 4.

Moreover, in this non-limiting embodiment, during the winding of the tape with the embedded roller **4** on the spirally grooved rod, the distances between the turns of the braid **3** and the place of winding the tape with the embedded roller **4** relative to the mouthpiece is controlled by the profiled wheel **11** applied to the braid **3,** as illustrated in Fig. 5.

Wherein, the method according to the invention is a continuous process, therefore, as early as in the step of winding the tape with the embedded roller **4** on the spirally grooved rod **7,** the curing of the produced tube **1** is conducted. In this embodiment, a cold water bath is used to cure the material. In the meantime, the tube **1** is slid from the grooved rod **7,** turning constantly and advancing in the axial direction. The tube **1** is dried and cut to a predetermined length by integrated tooling, wherein the predetermined lengths are cut from the continuously produced tube **1.**

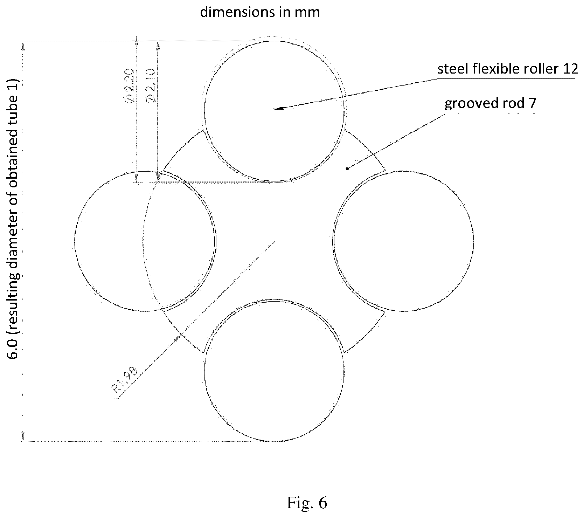

The method of the invention allows for the production of thin-walled spiral medical tubes **1** with an inside diameter <10 mm. Wherein, the diameter of the obtained tube **1** depends on the diameter of the grooved rod **7,** the diameter of the steel flexible rollers **12** and the diameter and depth of the grooves in the rod **7.** This embodiment results with the tube **1** with the internal diameter of 6 mm, the radius **R** of the grooved rod **7** is of 1.98 mm, while the diameter of the steel flexible rollers **12** is approximately 2.10-2.20. Wherein, the mutual arrangement of the rollers **12** and the grooved rod **7** is illustrated in Fig. 6.

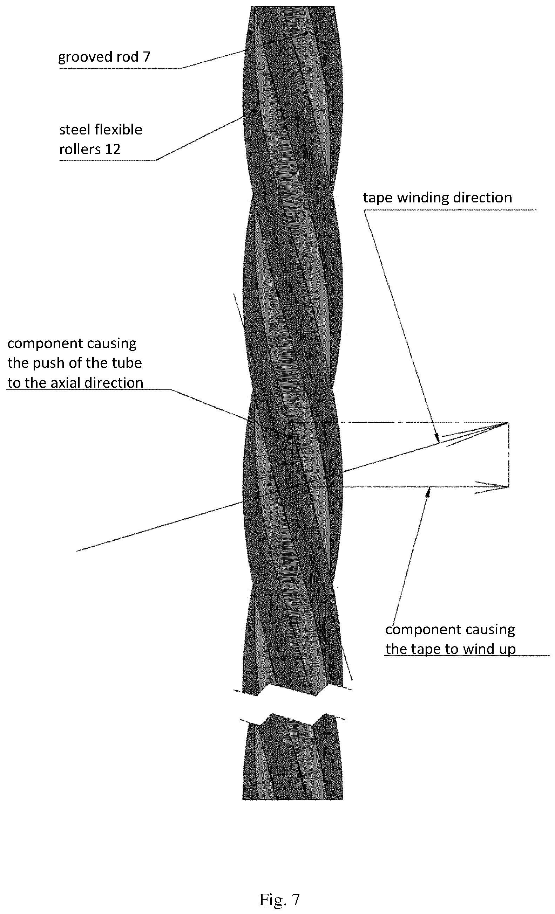

The parameter influencing the pitch of the spiral of the obtained medical spiral tube **1** is the pitch of the spirals grooved in the rod **7** in which the steel flexible rollers **12** are guided. The greater this pitch, the more, considering a side view of the system, the steel flexible rollers **12** are arranged parallel to the axial direction and the greater the proportion of the component causing rotation as compared to the advance of the produced medical spiral tube **1.** As a result, the pitch of the braid **3** of the spiral **1** will be smaller and hence the braid **3** will be denser. The pitch of the braid **3** should be matched with the width of the tape with the embedded roller **4.** This is illustrated in Fig. 7.

### Example 2.

In this embodiment, the flexible spiral medical tube **1** according to the invention has an internal diameter of 6 mm and was made by the method of example 1. As indicated in Fig. 1 the tube **1** consists of a tubular portion **2** made of PVC "738" and a constant pitch spiral braid **3** made of PVC "918" which is embedded into the tubular portion **2** over its entire contact surface with the tubular portion **2.**

Subsequently, the tube **1** made in this way was compared with a medical tube known from the state of the art. As medical spiral tubes with an inner diameter of <10 mm are not available in the prior art, the reference product was a conventional tube with an inside diameter of 5.8 mm, outside diameter of 8.3 mm and weight of 37 grams per meter, which was made of the same material as the tubular portion **2** of the tube **1** according to the invention (i.e. PVC "738" with hardness in Shore scale A of '73).

On the other hand, the tube **1** according to the invention showed a weight of 19 grams per meter, therefore it was 49% lighter than the reference tube, while maintaining all the required parameters in terms of strength, flexibility, reliability and chemical and biological reactivity.

Smaller volume and weight parameters of the tube **1** according to the invention ensure a significant reduction in the amount of generated waste (which is particularly significant in the case of disposable medical products made of plastics, which are toxic waste intended for incineration) and energy expenditure on the transport of parcels of lower weight.

An additional advantage is an increase in the comfort of patients and medical personnel using the tube **1** according to the invention.

### Example 3.

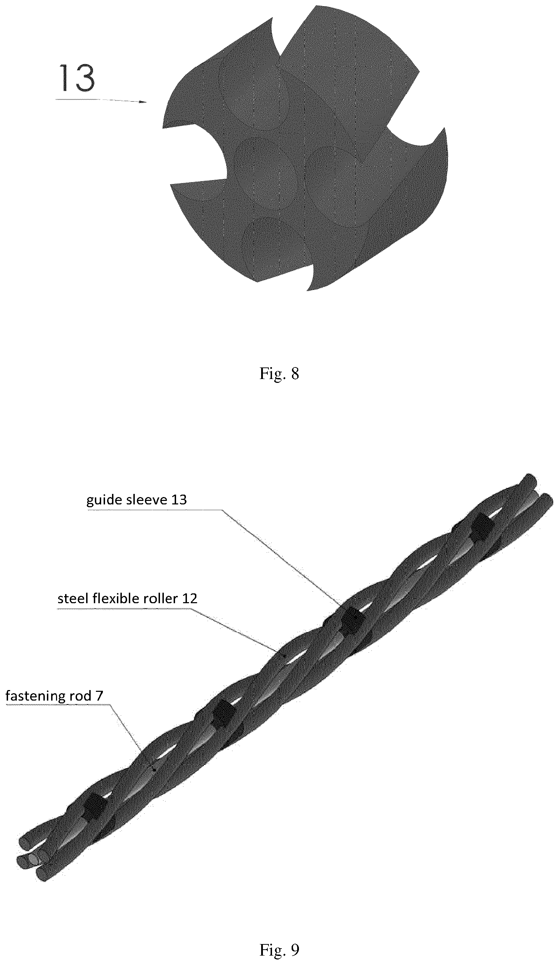

The method of producing a flexible thin-walled medical tube **1** according to the invention as in example 1, except that the rod **7** is a securing rod with a circular cross-section comprising guide sleeves **13** (Fig. 8), which are fixed on the mounting rod evenly at a predetermined distance. Wherein, as indicated in Figs. 8-9, said sleeves **13** are slidably superimposed on the securing rod and are characterised by grooves of an essentially cruciform shape (or a shape similar to slides cut from the spirally grooved rod of Fig. 3) which provides recesses for guiding steel flexible rollers **12.** The rod **7** in the variant of a circular rod with guide sleeves **13** and steel rollers **12** superimposed thereon is illustrated in Fig. 9. The use of the rod **7** in a non-grooved form with additional sleeves makes it possible to adjust, within a certain range, the winding parameters of the tape with an embedded roller **4** forming a medical spiral tube by shifting the sleeves **13** relative to each other.

### Example 4.

In this embodiment, for the production of a flexible thin-walled medical tube **1** according to the invention illustrated in Fig. 1, two extruders (constituting devices known from the state of the art) were used, loaded with two different materials, including the first material which was intended to produce the tubular portion **2** of the tube **1,** and the second material which was intended to produce the spiral braid **3.** Wherein, in accordance with the invention, the second used material has a higher hardness than the first material. The first material was extruded from the first mouthpiece **9a** connected to the first extruder, while the second material was extruded from the second mouthpiece **9b** connected to the second extruder. The mouthpiece **9a** of the first extruder is shaped as the cross-section of the flat and thin tape **5** and the mouthpiece **9b** of the second extruder is shaped as the cross-section of the roller **6.**

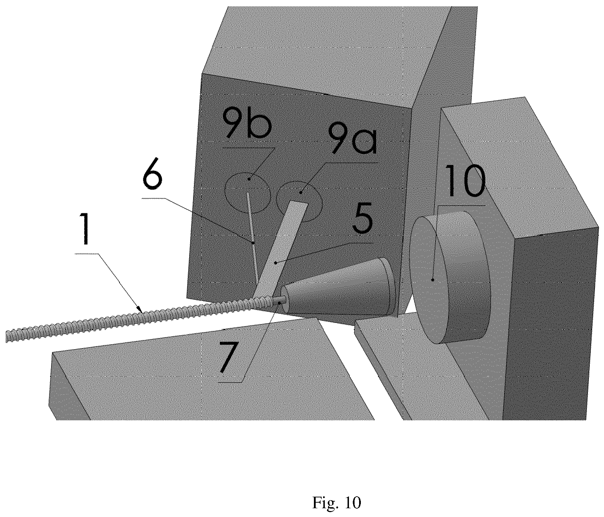

Subsequently, the flat and thin tape **5** and the roller **6** are wound on a spirally grooved rod **7** fixed in the gear **10.** The flat and thin tape **5** and the roller **6** connect to one another (i.e. the roller **6** is embedded into the tape **5),** simultaneously with their winding on said rod **7,** as illustrated in Fig. 10. Wherein, the edges of the flat and thin tape **5** constituting the component of the tape **4** are overlapped and fused together. Moreover, while the flat and thin tape **5** and roller **6** are wound on the rod, the produced tube **1** is cured, and then slid off the rod.

## Claims

1. A method of producing a flexible spiral medical tube consisting of a tubular portion and a braid, **characterised in that** it comprises the following steps:
a) filling a first extruder with a first material and a second extruder with a second material having a hardness greater than the first material;
b) mixing the materials in one mixing head (8) connected to the first and the second extruder;
c) forming a first material tape (5) and a second material roll (6) in the mixing head (8) of the extruders;
d) obtaining a tape with an embedded roller (4) by extruding both materials through a shaped mouthpiece (9);
e) winding the tape with the embedded roller (4) obtained in step d) on a rod (7) fixed in the gear (10), which is a spiral grooved rod, in recesses of which steel flexible rollers (12) are guided, or on a circular cross-section securing rod containing substantially cruciform guide sleeves (13) equipped with recesses for guiding flexible steel rollers (12), wherein the flexible rollers (12), transmitting the torsional moment from a motor of a gear (10) simultaneously push the wound tape with the embedded roller (4) in the axial direction, creating a spiral, wherein the edges of the tape with the embedded roller (4) are overlapped and fused together, and while the tape with the embedded roller (4) is wound on the rod (7), a curing of the produced tube (1) is carried out, which then slid off the rod (7).

2. The method according to claim 1, **characterised in that** in step e) a spirally grooved rod (7) with an almost cruciform cross-section is used.

3. A method according to any of the preceding claims from 1 to 3, **characterised in that** in step e), during the winding of the tape with an embedded roller (4) on a rod (7), the distances between the turns of the braid (3) and the place of winding the tape with the embedded roller (4) relative to the mouthpiece (9) of the extruder head are controlled by the profiled wheel (11) applied to the braid (3).

4. A method of producing a flexible spiral medical tube consisting of a tubular portion and a braid, **characterised in that** it comprises the following steps:
a) filling a first extruder with a first material and a second extruder with a second material having a hardness greater than the first material;
b) extruding a tape (5) of the first material through a shaped mouthpiece of a first mixing head (9a) with a shape of the tape (5) cross-section and extruding the roller (6) of the second material by extruding through a shaped mouthpiece of a second mixing head (9b) with a shape of the roller cross-section;
c) winding the tape (5) and the roller (6) obtained in step d) on the rod (7) fixed in the gear (10), which is a spirally grooved rod, in the recesses of which steel flexible rollers (12) are guided, or on a circular cross-section securing rod containing substantially cruciform guide sleeves (13) equipped with recesses for guiding flexible steel rollers (12), wherein when being wound on the rod (7), the tape (5) connects to the roller (6) by fusing the roller into the tape (5), while flexible steel rollers (12) transmit the torsional moment from a motor of the gear (10) and simultaneously push the wound tape with the embedded roller (4) in the axial direction, creating a spiral, wherein the edges of the produced tape with the embedded roller (4) are overlapped and fused together, and while the tape with an embedded roller (4) is wound on the rod (7), a curing of the produced tube (1) is carried out, which then slid off the rod (7).

5. The method according to claim 4, **characterised in that** the first head and the second head share a common housing.

6. The method according to claim 4 or 5, **characterised in that** in step e) a spirally grooved rod (7) with an almost cruciform cross-section is used.

7. A method according to any of the preceding claims from 4 to 6, **characterised in that** in step c), during the winding of the tape (5) and the roller (6) on the rod (7) with the simultaneous embedding of the roller (6) into the tape (5), the distances between the turns of the braid (3) and the place of winding the tape with the embedded roller (4) relative to the mouthpiece (9a and 9b) is controlled by the profiled wheel (11) applied to the braid (3).

8. A flexible spiral medical tube consisting of a tubular portion and a braid, **characterised in that** its internal diameter < 10 mm; the braid (3) is fused into the tubular portion (2) over its entire contact surface with the tubular portion (2); the tubular portion (2) is made of the first material and the braid (3) is made of the second material; wherein the hardness of the second material is higher than that of the first material.

9. The tube according to claim 8 **characterised in that** its inner diameter is 6 mm.

10. The tube according to claim 8 or 9 **characterised in that** the first material is selected from the group consisting of polyvinyl chloride (PVC), thermoplastic elastomers (TPE), polyethylene (PE).

11. The tube according any of the preceding claims from 8 or 10 **characterised in that** the first material is selected from the group consisting of polyvinyl chloride (PVC), thermoplastic elastomers (TPE), polyethylene (PE).

12. The tube according to any of the preceding claims. from 8 to 11, **characterised in that** the braid pitch (3) is constant.
